# EUROPEAN PATENT SPECIFICATION

(11) **EP 4 586 897 B1**
(45) Date of publication and mention of the grant of the patent: **12.08.2026**
(21) Application number: 23761156.1
(22) Date of filing: 28.08.2023
(51) Int. Cl.: A61B 5/0205, A61B 5/11, A61B 5/00

(54) **VITAL SIGN DETECTION DEVICE**
VORRICHTUNG ZUR ERKENNUNG VON LEBENSZEICHEN
DISPOSITIF DE DÉTECTION DE SIGNES VITAUX

(30) Priority: 16.09.2022 EP 22196077
(43) Date of publication of application: 23.07.2025
(73) Proprietor: Signify Holding B.V., 5656 AE Eindhoven (NL)
(72) Inventor: VAN DE SLUIS, Bartel, Marinus, 5656 AE Eindhoven (NL); DELNOIJ, Roger, Peter, Anna, 5656 AE Eindhoven (NL)
(74) Representative: Verweij, Petronella Daniëlle
(86) International application number: PCT/EP2023/073477
(87) International publication number: WO 2024/056353

(56) References cited:
- US-A1- 2019 117 091
- US-A1- 2022 155 737
- US-A1- 2022 218 224

## Description

### FIELD OF THE INVENTION

The current invention is directed to a vital sign detection device configured to operate in an environment frequented by multiple subjects, wherein each subject is a user of a univocally associated portable device, to a lighting device, to a lighting arrangement, to a method of associating vital sign data with a user of a portable device and with a computer program.

### BACKGROUND OF THE INVENTION

US 2019/0117091 A1 presents a technique for a monitoring device for remote monitoring of at least one vital sign of a living subject. The technique includes capturing one or more sensor signals that are descriptive of respective characteristics of a body of the subject from a distance, deriving, on basis of said one or more sensor signals, at least a first biometric signal that is descriptive of a second predefined vital sign of said subject for transmission to a server device, detecting presence of another monitoring device that is operating to derive a first biometric signal that is descriptive of a first predefined vital sign of the same subject for transmission to the server device, and controlling derivation of said first biometric signal in the detected another monitoring device in response to detecting said another device.

### SUMMARY OF THE INVENTION

Current technologies such as, for example, radar sensing, enable the acquisition or detection of personal vital sign data by a remote device, namely a vital sign detection device, at a specific distance range. However, if multiple subjects make use of the space (simultaneously or subsequently), the personal vital sign data is not univocally associated with a subject.

Therefore, it would be beneficial to enable the use of a vital sign detection device in an environment with multiple subjects.

The invention is defined by the appended claims.

According to a first aspect of the present invention, a vital sign detection device is disclosed. The vital sign detection device is configured to operate in an environment frequented by multiple subjects, wherein each subject is a user of a univocally associated portable device, said vital sign detection device having a vital sign detection unit configured to remotely detect vital sign data indicative of vital signs of a subject from the multiple subjects located in a vicinity of the vital sign detection device. Remotely, in the frame of this disclosure is to be understood as "from a distance; without physical contact". The vital sign detection device of the first aspect of the invention further comprises:
- a detection unit configured to detect presence of a portable device associated with a user, wherein the portable device is associated with the user for dedicated use or includes the user profile;
- a distance determination unit configured to determine a distance amount indicative of a distance between the portable device and the subject; and
- a data association unit configured to, upon determining that the distance amount matches a predetermined distance criterion, to associate the vital sign data of the subject to the user of the corresponding portable device, such that the detected vital sign data is linked or otherwise associated with the user of the portable device.

Thereby, the vital sign detection device, using a predetermined distance matching criterion, is advantageously configured to associate a detected vital sign to a user of a portable device that is also detected by the vital sign detection device. The portable device is in turn associated with the user. Thus, vital sign data is associated with a user via the detection of a portable device (i.e. a portable device that is capable of being detected in any particular way by the vital sign detection device), and a distinction between vital sign data detected from a different subject is enabled.

In the following, embodiments of the vital sign detection device of the first aspect of the invention will be disclosed.

In an embodiment, the distance matches the distance criterion, or the distance criterion is met, when the distance amount is below a predetermined threshold amount. In another embodiment, the distance criterion is additionally or alternatively met, when the portable device is located at a predetermined location with respect to the vital sign detection device, for instance at a docking or charging station.

In an embodiment, the portable device is a communication device, such as, but not limited to, a mobile phone, a tablet, a smart watch, a wearable device, a camera, etc., which is configured to provide communication signals in accordance with a suitable wireless communication protocol, and which is associated with a particular user or has an active user profile and that is configured to share data indicative of the user or user profile with the vital sign detection device.

In another embodiment, the vital sign detection device is configured to check if portable devices are detected within a remote sensing range of the vital sign detection device by detecting RF-signals provided by the portable devices in the proximity. In another embodiment, the detection unit is alternatively or additionally configured to detect portables devices by determining whether the portable devices are docked at a specific location. In yet another embodiment, the detection unit is configured to, using ascertained or received image data, detect the presence of a portable device.

In a particular embodiment, the vital signs that can be detected by the vital sign detection unit include one or more of heart beat, respiration, rapid-eye-movement, head movement, torso movement and limb movement. Preferably, the vital signs detected are indicative of a quality of sleep. Detectable vital signs preferably include micro movements of the body of the subject. Micro movements may be related to body movements caused by heart beats and/or respiration. Vital sign data such as heart rate or respiration patterns can be derived from said movements. Other body movements, when the subject is asleep, can be indicative of the subject's sleep quality and/or duration.

In an embodiment, the vital sign detection unit comprises a radiofrequency sensor and/or an optical sensor. Preferably the radiofrequency sensor is a radar sensor, in particular a radar sensor configured to detect micro movements of a subject, for example at a distance or a range from a few centimeters to a few meters (e.g., from 30 cm to 300cm). Vital sign sensing, for instance of breathing or heartbeat can be performed with millimeter accuracy and the accuracy is expected to increase in the future. Preferably, the radar sensor is a frequency modulated continuous wave (FMCW) radar, where phase information is used to determine the distance or range. Alternatively, the radar sensor may be implemented as a pulse radar, a UWB radar or as any generic time-of-flight radar sensor. The time-of flight sensor's operational principles are the same for electromagnetic (radar, laser etc.) and active acoustic sensing. Time-of-flight is the principle mode of operation for most radar, laser and active acoustic devices. This technique uses the time between the transmission of a pulse and the reception of an echo. The time is measured to provide range or distance information. Typically, the source of radiation is fed to a transmit antenna that is tuned to the characteristics of the target and preferably matched to the impedance of the medium to maximize coupling and efficiency. The signal is preferably radiated by a directional antenna to increase the energy on target. The signal impacts on the target of interest which results in a change in impedance, which in turn results in re-radiation or scattering, which can be isotropic, directional or random. A small percentage of the signal power is received at a receiver and converted to an electrical signal suitable for amplification and detection. In another embodiment, the radiofrequency sensor may additionally or alternative include a WiFi-sensing unit configured to perform WiFi sensing of the vital signs. WiFi sensing (also referred to as WLAN sensing) uses WiFi signals to detect events or changes in an environment, such as those caused by motion. It is also used for gesture recognition and biometric measurements such as breathing and heartbeat. Typically, WiFi sensing is based on a combination of WiFI and radar sensing technologies cooperating to enable usage of the same WiFi transceiver hardware and RF-spectrum for signal communication and sensing. Typically, movement or micro movements are detected by analyzing frequency disturbances in the WiFi signal, which is typically provided by means of orthogonal frequency-division multiplexing (OFDM).An optical sensor, such as a camera or a camera array is additionally or alternatively used in another embodiment to detect movements or micro movements associated with a vital sign, based on detected motion in the captured camera images.

In another embodiment, the vital sign detection unit actively detects vital signs of the subject and generates vital sign data indicative thereof. Additionally, or alternatively, the vital sign detection unit is in signal communication with other vital sign detector and receive the vital sign data from said vital sign detectors.

In another embodiment, which may include any of the technical features described above, the detection unit is further configured to detect a state of the portable device. In this particular embodiment, the vital sign detection unit is further configured to detect the vital sign data of the subject in dependence on the detected state of the portable device. For example, in an embodiment, the detection unit is configured to detect if the portable device is docked in a docking station, for instance, for charging, or its orientation (if the portable device is lying flat) or its movement or lack thereof (if the portable device is static). Further, the detection unit can be advantageously configured to ascertain whether the portable device is currently in a quiet mode, do-not-disturb mode, sleep mode, concentration mode, etc. In an embodiment, the state of the portable device is configured to trigger the detection of the vital sign data of the subject and the association, in accordance to the distance matching criterion, of the subject with the user of the portable device.

Additionally, or alternatively, in an embodiment, the detection unit is further configured detect a state of the user of the portable device. In this particular embodiment, the vital sign detection unit is additionally or alternatively configured to detect the vital sign data in dependence on the detected state of the user. For example, the detection unit is configured to ascertain a state of the user of the portable device by analyzing the current light settings in a room. An activation of a desk lamp may be indicative of the user starting a productive task, the deactivation of the bedroom lights may be indicative of the user going to sleep. Also data provided by the portable device can be indicative of the state of the user, namely of whether the user is active, or sitting down, or lying down. In an embodiment, the state of the user of the portable device is configured to trigger the detection of the vital sign data of the subject and the association, in accordance to the distance matching criterion, of the subject with the user of the portable device.

For instance, in an embodiment, detection of vital signs, preferably those associated with a quality of sleep, are triggered only when the state of the portable device is indicative -according to predetermined parameters- of the user having gone to sleep. In a room where more than one subject sleeps, the vital signs are associated with the respective subject based on the distance between the subject and the portable device (e.g. a mobile phone or a smart watch).

In a preferred embodiment, which may include any of the technical features discussed above, the distance determination unit is configured to determine a first relative position of the portable device, a second relative position of the subject, and using the determined first relative position and the second relative position, to determine the distance between the portable device and the subject. In particular, the distance determination unit is configured to determine the distance using a radiofrequency (RF) signal based positioning technique. RF signal based positioning techniques and related systems rely on transmission and reception of radio-frequency signals for target position determination. A particular system can make use of one or more signal parameters, including, but not restricted to the time-of-arrival (TOA), the time-difference-of-arrival (TDOA), the received signal strength (RSS), the received power profile, the angle-of-arrival (AOA), or the received signal phase. To achieve efficient signal transmission and/or reception, careful signal design must be performed. The choice of signaling methods will greatly affect the performance of positioning systems. When designing signals, a number of issues needs to be considered, including positioning accuracy, power consumption, frequency bandwidth, interference, cost, and policy compliance.

In another embodiment, the vital sign detection device further comprises a data merging unit configured to combine additional vital sign data that has been detected or otherwise acquired by the portable device with the detected vital sign in a user profile, when the association between the subject and the user has been established. In a particular development, the data merging unit comprises a data reception unit for receiving the additional vital sign data from the portable device. In another embodiment the data merging unit comprising a data provision unit for providing the vital sign data to the portable device, for combining both vital sign data in the portable device. In another embodiment, the data merging unit is configured to provide the vital sign data and/or the additional vital sign data to a cloud-based service. This is particularly advantageous for users that have a portable device that is also configured to ascertain vital signs, or activity data, such as a mobile phone, a smart watch or a wearable device. The vital sign data provided by the portable device may or may not be of the same type of that vital sign data detected by the vital sign detection device. The vital sign data provided by the portable device and that detected by the vital sign detection device may be integrated or combined into a user profile, namely an activity profile associated with the user of the portable device. Conversely, in another embodiment, the vital sign reception device is configured to, upon having established an association between the subject and the user of the portable device, to provide the vital sign data or data indicative thereof to the portable device for integration or combination of both sources of vital sign data (e.g. activity data) into a single user profile.

In another embodiment, the vital sign detection device further comprises a data provision unit configured to provide, to the portable device, connection data indicative of a successful association between the subject and the user of the portable device. The connection data can advantageously be used to inform the user that an association has been established. Also, the user may confirm or decline such association. Also, the data provision unit may be additionally or alternatively configured to provide the connection data to another device, such as a lighting device, to provide a visual indication (e.g. blinking, glowing, etc.,) that the association has been established and/or that vital sign data is being detected.

In a particular embodiment, the vital sign detection device further comprises a docking station for charging the portable device. In particular, in this embodiment, the detection unit is connected to the docking station and is further configured to detect the presence of the portable device when the portable device is docked at the docking station

According to a second aspect of the present invention, a lighting device is disclosed. The lighting device of the second aspect comprises a vital sign detection device according to the first aspect of the invention, and thus shares its advantages.
In a preferred embodiment, the lighting device is, for instance, a desk lamp, a bedroom or bedside lamp, a wake up light, a hospital room lamp, an elderly daylight lamp, a shower cabin lamp. Alternatively, the vital sign detection device can be integrated into any other device of a lighting arrangement, in particular a wirelessly controllable lighting arrangement, such a sensor (presence, light, movement, etc.,) or a switch. In any case, the antennas of the device should preferably be directed towards typical subject or user positions, such as a desk, a bed, a shower cabin, etc.

In a particular embodiment, the lighting device further comprises a docking station for charging the portable device. In particular, in this embodiment, the detection unit is connected to the docking station and is further configured to detect the presence of the portable device when the portable device is docked at the docking station.

A third aspect of the present invention is formed by a lighting arrangement, in particular a wirelessly controllable lighting arrangement. The lighting arrangement comprises a vital sign detection device according to the first aspect of the invention, which may be integrated in a lighting device to form a lighting device in accordance with the second aspect of the invention. The lighting arrangement of the third aspect also comprises one or more lighting devices. The operation state of the one or more lighting devices is controllable based on one or more of the vital sign data, a state of the portable device and a state of the user. For instance, vital sign data indicative of sleep patterns is used in an embodiment to control the lighting devices in a wake-up phase. Also, a lighting device, in particular a lighting device comprising the vital sign detection device is controlled, in an embodiment, to provide a visual indication indicative of the current vital sign sensing status. For instance, after docking a portable device, the lighting device can be instructed to blink or glow to indicate that a vital sign sensing phase is beginning or will shortly begin. Additionally, the portable device may be instructed to show a notification or confirmation message to indicate that the portable device, and therefore the user, has become associated with the vital sign detection device.

According to a fourth aspect of the present invention, it is disclosed a method of associating vital sign data of a subject to a user of a portable device in an environment frequented by multiple subjects, wherein each subject is the user of an univocally associated portable device. The method comprises:
- acquiring vital sign data indicative of vital signs of a subject from the multiple subjects;
- detecting presence of a portable device associated with a user;
- determining a distance between the portable device and the subject; and
- upon determining that the distance between the portable device and the subject matches a predetermined distance criterion, associating the subject to the user of the corresponding portable device.

The method of the fourth aspect thus shares the advantages of the vital sign detection device of the first aspect of the invention.

In an embodiment, the method also comprises controlling operation of a lighting device in dependence on the detected vital sign data.

An additional, fifth, aspect of the invention is formed by a computer program comprising instructions which, when the program is executed by a vital sign detection device of the first aspect of the invention, cause the vital sign detection device to carry out the method of the fourth aspect of the invention.

The invention is solely defined by the scope of the appended claims.

### BRIEF DESCRIPTION OF THE DRAWINGS

In the following drawings:
Fig. 1 shows a schematic block diagram of an exemplary vital sign detection device in accordance with an embodiment of the invention;
Fig. 2 shows a schematic block diagram of an exemplary lighting arrangement comprising a lighting device having a vital sign detection device in accordance with another embodiment of the invention; and
Fig. 3 shows a flow diagram of an exemplary method of associating vital sign data to a user of a portable device in accordance with an embodiment of the invention.

### DETAILED DESCRIPTION OF EMBODIMENTS

Techniques like radar-based sensing or optical sensing enable the remote (i.e. from a distance, without physical contact) detection of personal vital sign data by a remote device, referred to as vital sign detection device, by detecting for instance micro movements at a specific distance range. However, if multiple subjects make use of the same space (simultaneously or subsequently), the personal vital sign data should be associated with the corresponding subject.

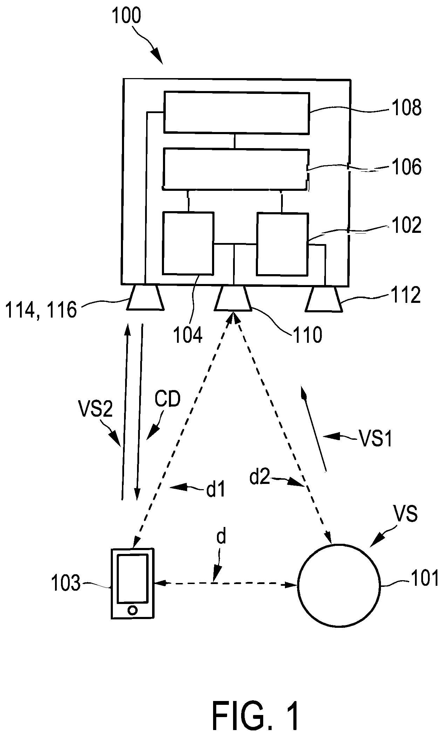

Fig. 1 shows a schematic block diagram of an exemplary vital sign detection device 100. The vital sign detection device 100 includes a vital sign detection unit 102 that is configured to remotely detect vital sign data VS1 indicative of vital signs VS of a subject 101 that is located in a vicinity (e.g. in the same room) of the vital sign detection device 100. Detectable vital signs include, but are not limited to, breathing, heart beating, other micro movements such as rapid eye movement, and movements of head, torso and/or limbs of the subject. Vital sign data refers to data indicative of said vital sign and include, for example, heart rate, breathing rate, sleep phase, and quality and/or duration of sleep.

Micromovements may related to bodily movements caused by heart beats and/or respiration. The vital sign data may be a derivative of those heartbeat or respiration patterns, such as a heart rate or respiration rate. Further vital signs include those associated with sleep movements, such as REM or body movements performed when the subject is asleep, which provide an indication of a user's sleep duration and sleep quality. In a similar way, concentration levels (e.g., when the subject is sitting at a desk) may be derived from body movements. The vital signs are preferably detected using a FMCW radar sensor 110. Additionally, or alternatively, a vision or optical sensor 112 such as a camera or camera array, can be used to detect vital signs, for example based on detected movements or micro movements in captured camera images. The vital sign detection device 100 is exemplary shown as a static device. However, a portable device is also possible. In any case, the vital sign detection unit 102 should be directed to typical subject positions, i.e., positions in the surrounding space or room where the subject is expected to stay a longer time, such as a desk, a bed, a shower cabin, a water closet, etc.

The vital sign detection device of Fig. 1 further comprises a detection unit 104 that is configured to detect presence of a portable device 103 associated with a user 101. Exemplary portable devices include smartphones, smart watches, tables, cameras or any other device which is associated with a user for dedicated use or includes a user profile. The dedicated use may comprise personal or professional use of the user. A distance determination unit 106 is configured to determine a distance amount d indicative of a distance between the portable device 103 and the subject 101. Further, a data association unit 108 is configured, upon determining that the distance amount d indicative of a distance between the portable device 103 and the subject 101 matches a predetermined distance criterion, e.g., is below a predetermined threshold amount dₜₕ, to associate the vital sign data VS1 of the subject 101 to the user of the portable device 103. In the case that the relative position of the portable device matches within a predetermined distance range, with that where the vital signs have been detected, the detected vital sign data will be associated with the user of the portable device 103.

Preferably, the detection unit 104 is further configured to detect a state of the portable device and/or a state of the user of the portable device. The vital sign detection unit 102 is then further configured to detect the vital sign data VS1 of the subject 101 in dependence on the detected state of the portable device 103 and/or the detected state of the user. For instance, it could be detected that the subject or the user is sitting or lying down, or that the portable device has changed its orientation and/or has becomes static. It could also be detected that the portable device is being placed down (horizontal), put in/on a charger or charging dock. In addition, the state of the user may be derived based on external parameter such as a current light setting of the room. For instance, a desk lamp may be activated (indicating that the user starts a productivity task) or bedroom lights may be switched off (indicating user going to sleep).

In an exemplary vital sign detection device, such as device 100 of Fig.1, the distance determination unit 106 is configured to determine a relative position of the portable device or a first relative distance d1 or range between the vital sign detection device 100 and the portable device 103. This is preferably done using a RF signal based positioning technique as readily known by the person skilled in the art. Further, the distance determination unit is also configured to detect a relative position (range) or a second relative distance d2 at which the vital sign-related movements or micro movements are being detected. The knowledge of the relative positions or of the first and the second relative distances d1 and d2 enable a determination of the distance amount d indicative of the distance between the subject 101 and the portable device 103.

Also optionally, the vital sign detection device 100 further comprises a data reception unit 114 that is configured to receive additional vital sign data VS2 from the portable device 103. Preferably, the additional vital sign data VS1 is combined with the detected vital sign data VS1 in a user profile when the association between the subject and the user has been established. This is particularly advantageous in cases where the portable device has a vital sign tracking unit or an activity tracking unit for ascertaining or tracking vital signs or activity profiles. The portable device (e.g. phone, wearable) is also configured to detect vital sign data and the this data may be combined with that detected by the vital sign detection device. Alternatively a handover is made from the portable device to the vital sign detection device 100, in particular once the portable device is put in a specific state, e.g. a watch that is taken off, or a phone that is put down or docked at a charger. It is also possible that a pre-defined relative user position (e.g. a bed or desk position) and/or portable device state (e.g. static) is machine-learned or user-configured for a docked personal device (e.g. at bedside table). Upon reaching this position and/or state, the vital sign detection device 100 may be configured to start detection of vital sign data. In this case, the portable device may be placed at a first relative position or distance d1, while the vital signs VS may be detected at a nearby second relative position or distance d2.

In addition, additional vital sign data VS2 gathered by the portable device (e.g., heart rate, breathing rate, movements) may be used to assist or calibrate an interpretation of the vital sign data VS1 detected by the vital sign detection device 100, e.g., to better determine the sleep stage. In particular the additional data (e.g. additional vital sign data or activity data) provided by the portable device (e.g. smart watch) is used to provide long term information to the vital signs detection device 100. For instance, the additional data provided by portable device data is preferably used as a reference indicative of a user's normal heart rate or heart rate profile and the vital sign data sensed by the vital sign detection device 100 can be interpreted based on this reference data, e.g. to classify a user's sleep stage..

Also optionally, the vital sign detection device 100 further comprises a data provision unit 116 configured to provide, to the portable device 103, connection data CD indicative of a successful association between the subject 101 and the user of the portable device 103. The connection data may be used to instruct the portable device to shows a visual, acoustic or haptic notification to indicate that this portable device has become associated with the subject or has been detected by the vital sign detection device 100. The notification may include a request for the user to accept or reject the association.

More advanced vital sign detection devices (bundle) devices may be further configured gather additional health data, such as measuring body temperature, or measuring the user's skin tone for given lighting conditions/settings.

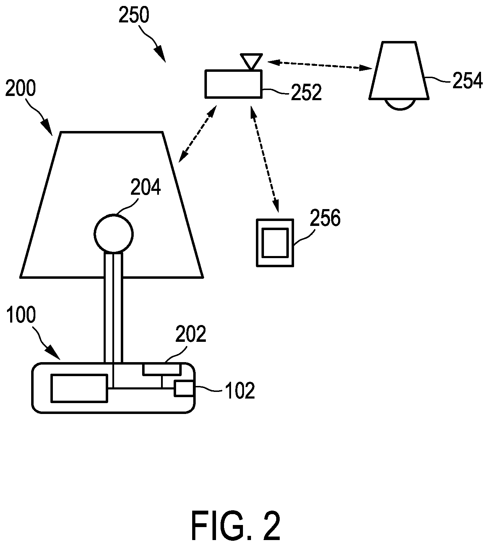

Fig. 2 shows a schematic block diagram of an exemplary lighting arrangement 250 comprising a lighting device 200 having a vital sign detection device, for instance, but not necessarily, vital sign detection device 100 of Fig. 1.

The lighting arrangement 250 is exemplarily a wirelessly controllable lighting arrangement that comprises a bridge or controller 252 in signal communication with a plurality of devices that include a first lighting device 200, a second lighting device 254 and a switch 256 for controlling operation (on/off, lighting settings) of the lighting devices 200, 252 of the lighting arrangement. Lighting device 200 comprises a lighting unit 204. The vital sign detection device 100 is integrated into the lighting device 200, which is here represented as a bedside lamp. The lighting device advantageously comprises a docking station 202 suitable for docking or charging one or more portable devices. The docking station may include a connector and/or an induction charger. The detection unit of the vital sign detection device 100 is connected to the docking station 202 and is further configured to detect the presence of the portable device 103 when the portable device is docked at the docking station 202. Preferably, the detection of the vital sign data starts once the portable device has been detected. In this case, the portable device is at a different relative position compared to the user, so the vital sign detection device is preferably configured to detect the subject, i.e. his or her vital signs, at predetermined expected positions.

In examples such as that of Fig. 2, where the vital sign detection device 100 is integrated in a lighting device 200, the lighting device 200 may additionally be controlled to provide an indication about its current sensing state. For instance, after docking a portable device the lighting unit 204 may be instructed to briefly blink or glow to indicate that the vital sign detection device is about to start vital sign sensing. However, in other examples where the vital sign detection device is not integrated into the lighting device, the arrangement, and in particular the controller 252, may be configured to provide instructions to the lighting device to operate the lighting unit as explained above.

In particular, the operation state of the one or more lighting devices 200, 254 of the lighting arrangement 250 is controllable based on one or more of the vital sign data VS1,VS2, a state of the portable device 103 and a state of the user.

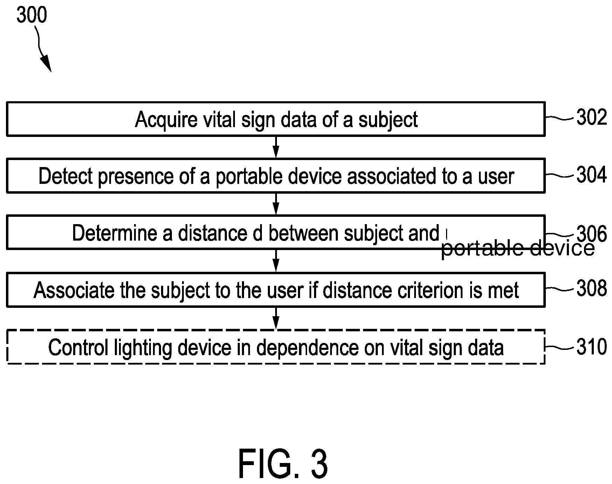

Fig. 3 shows a flow diagram of an exemplary method 300 of associating vital sign data to a user of a portable device in accordance with an embodiment of the invention. The method 300 comprises, in a step 302, acquiring vital sign data indicative of vital signs of a subject. The method also comprises, in a step 304, detecting presence of a portable device associated with a user. Further, the method comprises determining, in a step 306, a distance amount d indicative of a distance between the portable device and the subject. Upon determining that the distance amount d between the portable device and the subject matches a predetermined distance criterion, for instance, is below a predetermined threshold amount dₜₕ, the method includes associating, in a step 308, the subject to the user of the portable device, such that the detected vital sign data is assigned to the user.

Optionally, as indicated by the dashed box, the method 300 may include, in a step 310, controlling operation of a lighting device in dependence on the detected vital sign data.

In other exemplary methods according to the invention, the order of the steps are varied with respect to the order shown in Fig. 3. For instance, the method includes the following steps in the corresponding order:
a) detecting a portable device nearby the vital sign detection device;
b) estimating a relative position (range) of the portable device;
c) acquiring vital sign data, e.g. based on micro movements detected by a suitable vital sign detection unit;
d) determining a relative position (range) at which the vital signs are detected
e) associating the detected vital sign data with the user of the portable device when a predetermined distance matching criterion is fulfilled; and,
f) optionally, controlling a lighting device based on the vital sign data.

Any other step order that results in an association between the subject and the user of the portable device falls under the scope of the present invention.

In summary, the invention is directed to a vital sign detection device having a vital sign detection unit configured to remotely detect vital sign data indicative of vital signs of a subject located in a vicinity of the vital sign detection device. The vital sign detection device further comprises a detection unit configured to detect presence of a portable device associated with a user, a distance determination unit configured to determine a distance amount indicative of a distance between the portable device and the subject, and a data association unit configured, upon determining that the distance amount matches a predetermined distance criterion, to associate the vital sign data of the subject to the user of the portable device. Thus, the vital sign detection device is suitable for use in spaces visited by multiple subjects.

Some other examples of the invention may comprise a monitoring device (100) having a monitoring unit (102) configured to remotely detect/monitor monitoring data indicative of a charateristic of a subject (101) located in a vicinity of the monitoring device, the monitoring device further comprising: a detection unit (104) configured to detect presence of a portable device (103) associated with a user (101); a distance determination unit (106) configured to determine a distance amount (d) indicative of a distance between the portable device and the subject; and a data association unit (108) configured, upon determining that the distance amount matches a predetermined distance criterion, to associate the monitoring data of the subject with the user of the portable device.

In this example, the portable device (103) may be associated with the user for dedicated use or includes the user profile. The characteristic may comprise a health-related characteristic such as fall-detection, vital signs detection, breathing detection, sleep monitoring etc., and/or an activity of the user such as exercise, cooking etc. Other health related measures and/or activities are not excluded.

It shall be understood that above example can also be combined with any other embodiment of the invention.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the processing of the detection signals for measuring the vital signs, the authentication, the control of the vital sign monitoring system, et cetera, performed by one or several units or devices can be performed by any other number of units or devices. These procedures, particularly the control of the vital sign monitoring system in accordance with the control method carried out can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope. It is understood that the invention is solely limited by the scope of the appended claims.

## Claims

1. Vital sign detection device (100) configured to operate in an environment frequented by multiple subjects, wherein each subject is a user of a univocally associated portable device, said vital sign detection device having a vital sign detection unit (102) configured to remotely detect vital sign data indicative of vital signs of a subject (101) from the multiple subjects located in a vicinity of the vital sign detection device, the vital sign detection device further comprising:
- a detection unit (104) configured to detect presence of a portable device (103) associated with a user (101), wherein the portable device (103) is associated with the user for dedicated use or includes the user profile;
- a distance determination unit (106) configured to determine a distance amount (d) indicative of a distance between the portable device and the subject; and
- a data association unit (108) configured, upon determining that the distance amount matches a predetermined distance criterion, to associate the vital sign data of the subject with the user of the corresponding portable device.

2. The vital sign detection device (100) of claim 1, wherein the detectable vital signs include one or more of heart beat, respiration, rapid-eye-movement, head movement, torso movement and limb movement.

3. The vital sign detection device of claim 1 or 2, wherein the vital sign detection unit (102) comprises a radiofrequency sensor (110) and/or an optical sensor (112).

4. The vital sign detection device (100) of any of the preceding claims, wherein the detection unit is further configured to detect a state of the portable device and wherein the vital sign detection unit is further configured to detect the vital sign data of the subject in dependence on the detected state of the portable device.

5. The vital sign detection device (100) of any of the preceding claims, wherein the detection unit is further configured detect a state of the user of the portable device and wherein the vital sign detection unit is further configured to detect the vital sign data in dependence on the detected state of the user.

6. The vital sign detection device (100) of any of the preceding claims, wherein the distance determination unit (106) is configured to determine a first relative position (d1) of the portable device, a second relative position of the subject, and using the determined first relative position and the second relative position, to determine the distance between the portable device and the subject.

7. The vital sign detection device (100) of any of the preceding claims, wherein the distance determination unit (106) is configured to determine the distance (d) using a radiofrequency signal based positioning technique.

8. The vital sign detection device (100) of any of the preceding claims further comprising a data merging unit (114) configured to combine additional vital sign data acquired by the portable device with the detected vital sign data in a user profile when the association between the subject and the user has been established.

9. The vital sign detection device of any of the preceding claims, further comprising a data provision unit (116) configured to provide, to the portable device (103), connection data (CD) indicative of a successful association between the subject (101) and the user of the portable device (103).

10. The vital sign detection device (200) of any of the preceding claims, further comprising a docking station (202) for charging the portable device, and wherein the detection unit (104) is connected to the docking station and is further configured to detect the presence of the portable device (103) when the portable device is docked at the docking station (202).

11. A lighting device (200) comprising a vital sign detection device (100) according to any of the preceding claims.

12. A lighting arrangement (250) comprising a vital sign detection device according to any of the claims 1 to 10, and one or more lighting devices (200, 254), and wherein the operation state of the one or more lighting devices is controllable based on one or more of the vital sign data, a state of the portable device and a state of the user.

13. Method (300) of associating vital sign data of a subject to a user of a portable device in an environment frequented by multiple subjects, wherein each subject is the user of an univocally associated portable device, the method comprising:
- acquiring (302) vital sign data indicative of vital signs of a subject from the multiple subjects;
- detecting (304) presence of a portable device associated with a user, wherein the portable device (103) is associated with the user for dedicated use or includes the user profile;
- determining (306) a distance amount indicative of a distance between the portable device and the subject; and
- upon determining that the distance matches a predetermined distance criterion, associating (308) the subject to the user of the corresponding portable device.

14. The method (300) of claim 13, further comprising
- controlling operation (310) of a lighting device in dependence on the detected vital sign data.

15. A computer program comprising instructions which, when the program is executed by a vital sign detection device according to claim 1, cause the vital sign detection device to carry out the method of claim 13 or 14.

## Patentansprüche

1. Vitalzeichenerfassungsvorrichtung (100), die konfiguriert ist, um in einer von mehreren Subjekten frequentierten Umgebung in Betrieb zu sein, wobei jedes Subjekt ein Benutzer einer eindeutig zugeordneten tragbaren Vorrichtung ist, wobei die Vitalzeichenerfassungsvorrichtung eine Vitalzeichenerfassungseinheit (102) aufweist, die konfiguriert ist, um Vitalzeichendaten aus der Ferne zu erfassen, die Vitalzeichen eines Subjekts (101) aus den mehreren Subjekten angeben, das sich in einer Nähe der Vitalzeichenerfassungsvorrichtung befindet, die Vitalzeichenerfassungsvorrichtung ferner umfassend:
- eine Erfassungseinheit (104), die konfiguriert ist, um ein Vorhandensein einer tragbaren Vorrichtung (103) zu erfassen, die einem Benutzer (101) zugeordnet ist, wobei die tragbare Vorrichtung (103) dem Benutzer für eine dedizierte Verwendung zugeordnet ist oder das Benutzerprofil einschließt;
- eine Abstandsbestimmungseinheit (106), die konfiguriert ist, um einen Abstandsbetrag (d) zu bestimmen, der einen Abstand zwischen der tragbaren Vorrichtung und dem Subjekt angibt; und
- eine Datenzuordnungseinheit (108), die konfiguriert ist, um bei einem Bestimmen, dass der Abstandsbetrag mit einem zuvor bestimmten Abstandskriterium übereinstimmt, die Vitalzeichendaten des Subjekts dem Benutzer der entsprechenden tragbaren Vorrichtung zuzuordnen.

2. Vitalzeichenerfassungsvorrichtung (100) nach Anspruch 1, wobei die erfassbaren Vitalzeichen eines oder mehrere von Herzschlag, Atmung, schneller Augenbewegung, Kopfbewegung, Rumpfbewegung und Gliedmaßenbewegung einschließen.

3. Vitalzeichenerfassungsvorrichtung nach Anspruch 1 oder 2, wobei die Vitalzeichenerfassungseinheit (102) einen Hochfrequenzsensor (110) und/oder einen optischen Sensor (112) umfasst.

4. Vitalzeichenerfassungsvorrichtung (100) nach einem der vorstehenden Ansprüche, wobei die Erfassungseinheit ferner konfiguriert ist, um einen Zustand der tragbaren Vorrichtung zu erfassen, und wobei die Vitalzeichenerfassungseinheit ferner konfiguriert ist, um die Vitalzeichendaten des Subjekts in Abhängigkeit von dem erfassten Zustand der tragbaren Vorrichtung zu erfassen.

5. Vitalzeichenerfassungsvorrichtung (100) nach einem der vorstehenden Ansprüche, wobei die Erfassungseinheit ferner konfiguriert ist, um einen Zustand des Benutzers der tragbaren Vorrichtung zu erfassen, und wobei die Vitalzeichenerfassungseinheit ferner konfiguriert ist, um die Vitalzeichendaten in Abhängigkeit von dem erfassten Zustand des Benutzers zu erfassen.

6. Vitalzeichenerfassungsvorrichtung (100) nach einem der vorstehenden Ansprüche, wobei die Abstandsbestimmungseinheit (106) konfiguriert ist, um eine erste relative Position (d1) der tragbaren Vorrichtung und eine zweite relative Position des Subjekts zu bestimmen, und um unter Verwendung der bestimmten ersten relativen Position und der zweiten relativen Position den Abstand zwischen der tragbaren Vorrichtung und dem Subjekt zu bestimmen.

7. Vitalzeichenerfassungsvorrichtung (100) nach einem der vorstehenden Ansprüche, wobei die Abstandsbestimmungseinheit (106) konfiguriert ist, um den Abstand (d) unter Verwendung einer auf einem Hochfrequenzsignal basierenden Positionierungstechnik zu bestimmen.

8. Vitalzeichenerfassungsvorrichtung (100) nach einem der vorstehenden Ansprüche, ferner umfassend eine Datenzusammenführungseinheit (114), die konfiguriert ist, um zusätzliche Vitalzeichendaten, die von der tragbaren Vorrichtung erlangt wurden, mit den erfassten Vitalzeichendaten in einem Benutzerprofil zu kombinieren, wenn die Zuordnung zwischen dem Subjekt und dem Benutzer hergestellt wurde.

9. Vitalzeichenerfassungsvorrichtung nach einem der vorstehenden Ansprüche, ferner umfassend eine Datenbereitstellungseinheit (116), die konfiguriert ist, um der tragbaren Vorrichtung (103) Verbindungsdaten (CD) bereitzustellen, die eine erfolgreiche Zuordnung zwischen dem Subjekt (101) und dem Benutzer der tragbaren Vorrichtung (103) angeben.

10. Vitalzeichenerfassungsvorrichtung (200) nach einem der vorstehenden Ansprüche, ferner umfassend eine Andockstation (202) zum Laden der tragbaren Vorrichtung, und wobei die Erfassungseinheit (104) mit der Andockstation verbunden ist und ferner konfiguriert ist, um das Vorhandensein der tragbaren Vorrichtung (103) zu erfassen, wenn die tragbare Vorrichtung an der Andockstation (202) angedockt ist.

11. Beleuchtungsvorrichtung (200), umfassend eine Vitalzeichenerfassungsvorrichtung (100) nach einem der vorstehenden Ansprüche.

12. Beleuchtungsanordnung (250), umfassend eine Vitalzeichenerfassungsvorrichtung nach einem der Ansprüche 1 bis 10 und eine oder mehrere Beleuchtungsvorrichtungen (200, 254), und wobei der Betriebszustand der einen oder der mehreren Beleuchtungsvorrichtungen basierend auf einem oder mehreren von den Vitalzeichendaten, einem Zustand der tragbaren Vorrichtung und einem Zustand des Benutzers steuerbar ist.

13. Verfahren (300) zum Zuordnen von Vitalzeichendaten eines Subjekts zu einem Benutzer einer tragbaren Vorrichtung in einer von mehreren Subjekten frequentierten Umgebung, wobei jedes Subjekt der Benutzer einer eindeutig zugeordneten tragbaren Vorrichtung ist, das Verfahren umfassend:
- Erlangen (302) von Vitalzeichendaten, die Vitalzeichen eines Subjekts aus den mehreren Subjekten angeben;
- Erfassen (304) des Vorhandenseins einer tragbaren Vorrichtung, die einem Benutzer zugeordnet ist, wobei die tragbare Vorrichtung (103) dem Benutzer für die dedizierte Verwendung zugeordnet ist oder das Benutzerprofil einschließt;
- Bestimmen (306) eines Abstandsbetrags, der einen Abstand zwischen der tragbaren Vorrichtung und dem Subjekt angibt; und
- bei dem Bestimmen, dass der Abstand mit einem zuvor bestimmten Abstandskriterium übereinstimmt, Zuordnen (308) des Subjekts zu dem Benutzer der entsprechenden tragbaren Vorrichtung.

14. Verfahren (300) nach Anspruch 13, ferner umfassend
- Steuern eines Betriebs (310) einer Beleuchtungsvorrichtung in Abhängigkeit von den erfassten Vitalzeichendaten.

15. Computerprogramm, umfassend Anweisungen, die, wenn das Programm von einer Vitalzeichenerfassungsvorrichtung nach Anspruch 1 ausgeführt wird, die Vitalzeichenerfassungsvorrichtung veranlassen, das Verfahren nach Anspruch 13 oder 14 durchzuführen.

## Revendications

1. Dispositif de détection de signes vitaux (100) configuré pour fonctionner dans un environnement fréquenté par de multiples sujets, dans lequel chaque sujet est un utilisateur d'un dispositif portable associé de manière univoque, ledit dispositif de détection de signes vitaux ayant une unité de détection de signes vitaux (102) configurée pour détecter à distance des données de signes vitaux indiquant des signes vitaux d'un sujet (101) parmi les multiples sujets situés à proximité du dispositif de détection de signes vitaux, le dispositif de détection de signes vitaux comprenant en outre :
- une unité de détection (104) configurée pour détecter une présence d'un dispositif portable (103) associé à un utilisateur (101), dans lequel le dispositif portable (103) est associé à l'utilisateur pour une utilisation dédiée ou comporte le profil utilisateur ;
- une unité de détermination de distance (106) configurée pour déterminer une quantité de distance (d) indiquant une distance entre le dispositif portable et le sujet ; et
- une unité d'association de données (108) configurée, après la détermination que la quantité de distance correspond à un critère de distance prédéterminé, pour associer les données de signes vitaux du sujet à l'utilisateur du dispositif portable correspondant.

2. Dispositif de détection de signes vitaux (100) selon la revendication 1, dans lequel les signes vitaux pouvant être détectés comportent un ou plusieurs parmi le rythme cardiaque, la respiration, des mouvements oculaires rapides, des mouvements de la tête, des mouvements du torse et des membres.

3. Dispositif de détection de signes vitaux selon la revendication 1 ou 2, dans lequel l'unité de détection de signes vitaux (102) comprend un capteur radiofréquence (110) et/ou un capteur optique (112).

4. Dispositif de détection de signes vitaux (100) selon l'une quelconque des revendications précédentes, dans lequel l'unité de détection est en outre configurée pour détecter un état du dispositif portable, et dans lequel l'unité de détection de signes vitaux est en outre configurée pour détecter les données de signes vitaux du sujet en fonction de l'état détecté du dispositif portable.

5. Dispositif de détection de signes vitaux (100) selon l'une quelconque des revendications précédentes, dans lequel l'unité de détection est en outre configurée pour détecter un état de l'utilisateur du dispositif portable, et dans lequel l'unité de détection de signes vitaux est en outre configurée pour détecter les données de signes vitaux en fonction de l'état détecté de l'utilisateur.

6. Dispositif de détection de signes vitaux (100) selon l'une quelconque des revendications précédentes, dans lequel l'unité de détermination de distance (106) est configurée pour déterminer une première position relative (d1) du dispositif portable, une seconde position relative du sujet, et pour déterminer, à l'aide de la première position relative déterminée et de la seconde position relative, la distance entre le dispositif portable et le sujet.

7. Dispositif de détection de signes vitaux (100) selon l'une quelconque des revendications précédentes, dans lequel l'unité de détermination de distance (106) est configurée pour déterminer la distance (d) à l'aide d'une technique de positionnement basée sur des signaux radiofréquence.

8. Dispositif de détection de signes vitaux (100) selon l'une quelconque des revendications précédentes, comprenant en outre une unité de fusion de données (114) configurée pour combiner des données de signes vitaux supplémentaires acquises par le dispositif portable avec les données de signes vitaux détectées dans un profil utilisateur lorsque l'association entre le sujet et l'utilisateur a été établie.

9. Dispositif de détection de signes vitaux selon l'une quelconque des revendications précédentes, comprenant en outre une unité de fourniture de données (116) configurée pour fournir, au dispositif portable (103), des données de connexion (CD) indiquant une association réussie entre le sujet (101) et l'utilisateur du dispositif portable (103).

10. Dispositif de détection de signes vitaux (200) selon l'une quelconque des revendications précédentes, comprenant en outre une station d'accueil (202) destinée à charger le dispositif portable, et dans lequel l'unité de détection (104) est connectée à la station d'accueil et est en outre configurée pour détecter la présence du dispositif portable (103) lorsque le dispositif portable est connecté à la station d'accueil (202).

11. Dispositif d'éclairage (200) comprenant au moins un dispositif de détection de signes vitaux (100) selon l'une quelconque des revendications précédentes.

12. Agencement d'éclairage (250) comprenant un dispositif de détection de signes vitaux selon l'une quelconque des revendications 1 à 10, et un ou plusieurs dispositifs d'éclairage (200, 254), et dans lequel l'état de fonctionnement du ou des dispositifs d'éclairage peut être commandé en fonction d'un ou de plusieurs parmi les données de signes vitaux, un état du dispositif portable et un état de l'utilisateur.

13. Procédé (300) pour l'association de données de signes vitaux d'un sujet à un utilisateur d'un dispositif portable dans un environnement fréquenté par de multiples sujets, dans lequel chaque sujet est l'utilisateur d'un dispositif portable associé de manière univoque, le procédé comprenant :
- l'acquisition (302) de données de signes vitaux indiquant les signes vitaux d'un sujet parmi les multiples sujets ;
- la détection (304) d'une présence d'un dispositif portable associé à un utilisateur, dans lequel le dispositif portable (103) est associé à l'utilisateur pour une utilisation dédiée ou comporte le profil d'utilisateur ;
- la détermination (306) d'une quantité de distance indiquant une distance entre le dispositif portable et le sujet ; et
- lorsqu'il est déterminé que la distance correspond à un critère de distance prédéterminé, l'association (308) du sujet à l'utilisateur du dispositif portable correspondant.

14. Procédé (300) selon la revendication 13, comprenant en outre
- la commande du fonctionnement (310) d'un dispositif d'éclairage en fonction des données de signes vitaux détectées.

15. Programme informatique comprenant des instructions qui, lorsque le programme est exécuté par un dispositif de détection de signes vitaux selon la revendication 1, amènent le dispositif de détection de signes vitaux à mettre en œuvre le procédé selon la revendication 13 ou 14.
